# EUROPEAN PATENT SPECIFICATION

(11) **EP 2 786 727 B1**
(45) Date of publication and mention of the grant of the patent: **21.06.2017**
(21) Application number: 12853451.8
(22) Date of filing: 28.11.2012
(51) Int. Cl.: A61F 2/38

(54) **ARTIFICIAL KNEE JOINT**
KÜNSTLICHES KNIEGELENK
ARTICULATION ARTIFICIELLE DU GENOU

(30) Priority: 28.11.2011 CN 201110385645
(43) Date of publication of application: 08.10.2014
(73) Proprietor: Beijing Naton Technology Group Co., Ltd, Beijing 100082 (CN)
(72) Inventor: QU, Tiebing, Beijing 100095 (CN); CHENG, Chengkung, Beijing 100095 (CN); SONG, Dayong, Beijing 100095 (CN); HOU, Lili, Beijing 100095 (CN)
(74) Representative: Bösl, Raphael Konrad
(86) International application number: PCT/CN2012/085436
(87) International publication number: WO 2013/078994

(56) References cited:
- EP-A1- 1 440 675
- CN-A- 102 006 840
- CN-A- 102 076 283
- CN-U- 202 335 945
- US-A- 5 549 686
- US-A- 6 123 729
- US-A1- 2005 209 701
- US-A1- 2005 209 701
- US-A1- 2009 319 049
- US-A1- 2010 016 979

## Description

### TECHNICAL FIELD

The present disclosure relates to a prosthesis applied to the human body, and in particular to a prosthesis having a structural function of a knee joint.

### BACKGROUND

Currently, the normal walking and flexion and extension of knee joints of many patients cannot be achieved due to the damage of muscles and cartilaginous tissues. In this case, prostheses are required to be implanted to replace the knee joints to help the patients to stand, walk and flex and extend normally again. Although the artificial knee joints have been widely applied, the traditional artificial knee joint is not meticulous enough when simulating the action of the human body, and hence the human body cannot act in position smoothly when using the prosthesis and often cannot act properly. Particularly when the flexion of the knee joint is simulated, the traditional artificial knee joint only pays attention to the flexion and extension between a femur and a tibia of the knee joint during the flexion but ignores the external rotation of the femur relative to the tibia during the flexion. Therefore, it is substantively very difficult to achieve proper flexion in position, and the prosthesis can even be damaged as the action does not comply with the physiological characteristic of the human body; moreover, even the femur and the tibia of the patient can be damaged as well, and hence the patient's condition can be worse.

However, the external rotation of the knee of the human body during the flexion must be smooth and the movement range cannot be too large, or else the patient would be hurt. At present, although the problem has been considered by those skilled in the art, an actual means for solving the problem cannot be put forward. In the process of the simulation of the external rotation of the femur relative to the tibia, not only the action must be smooth but also further external rotation must be stopped in time. If the external rotation cannot be stopped in time, the problem of dislocation will be caused and even the personal safety will be affected. Therefore, as no effective means can be provided so far, the prosthesis designer is afraid of this for exclusion of liability.

As known prior art, US2010/016979 A1 discloses a knee replacement system comprising a femoral component, a tibial bearing insert, a posterior cam, and a posterior camming portion, wherein a rear surface of the posterior camming portion comprises one single arc surface, and the posterior cam correspondingly comprises one single arc surface to cooperate with the arc surface of the posterior camming portion. Moreover, EP 1 440 675 A1 discloses a knee prosthesis comprising a femoral component, a tibial component, and a stabilizing mechanism hving a stabilizing compartment on the femoral component and a post disposed on the tibial component, wherein, on the outside of a rear surface of the post, the post has one single arc surface to face another single arc surface, i.e. a follower surface/anterior face of a follower extending transversely across the interior of the stabilizing compartment. Further, US 2009/319049 A1 discloses a knee replacement prosthesis comprising a femoral component and a tibial component, which tibial component comprises a tibial platform and a liner, wherein a central post is disposed on the liner, and a cam is disposed in the femoral component, and wherein -on the outside of a rear surface of the post- the post has a single arc surface. Furthermore, US 2005/209701 A1 discloses an artificial knee joint comprising a femoral component and a tibial component, wherein the femoral component comprises a cam, and the tibial component comprises a post, and wherein -on the outside of a rear surface of the post- the post has a single arc surface, i.e., the posterior surface of the post.

### SUMMARY

The objective of the present disclosure is to solve the problem of the prior art and provide an artificial knee joint, in which the structure is simple and the knee joint can not only perform the flexion motion but also achieve the external rotation of a femur relative to a tibia during the flexion and extension of the knee joint.

In order to achieve the objective, the present disclosure adopts the following technical solutions:

An artificial knee joint as defined in claim 1 comprises a femoral component and a tibial component cooperating with each other and applied to a femur and a tibia, respectively; the femoral component is provided with an intercondylar groove; a cam is disposed at the rear of the intercondylar groove; the tibial component is provided with a medial condyle supporting surface and a lateral condyle supporting surface which are disposed on the left side and the right side, respectively; an upright post is disposed between the medial condyle supporting surface and the lateral condyle supporting surface and cooperates with the intercondylar groove; a first arc surface is formed on the outside of a rear surface of the upright post; a second arc surface is formed on the rear surface of the upright post, on the inside of the first arc surface; the cam is correspondingly provided with a third arc surface and a fourth arc surface cooperating with the first arc surface and the second arc surface, respectively; the first arc surface can cooperate with the third arc surface to guide the external rotation of the femur relative to the tibia; and the second arc surface can cooperate with the fourth arc surface to limit the external rotation of the femur relative to the tibia.

Moreover, in view of the cross section, the curvature of the first arc surface is the same as that of the third arc surface; the curvature of the second arc surface is the same as that of the fourth arc surface; and the curvature of the second arc surface and the fourth arc surface is less than that of the first arc surface and the third arc surface.

Moreover, the first arc surface, the second arc surface, the third arc surface and the fourth arc surface are all circular surfaces.

Moreover, the radius of the first arc surface and the third arc surface is 3 to 10 mm; and the radius of the second arc surface and the fourth arc surface is 30 to 50 mm.

Moreover, an included angle between a straight line, which connects a center of a circle of the first arc surface and a juncture between the first arc surface and the second arc surface, and a straight line, which is in the fore-and-aft direction of the tibia, is 5 to 20 degrees.

Moreover, the upright post is asymmetrically disposed on the tibial component and is disposed on a postmedial portion of the tibial component, biased towards the lateral condyle supporting surface.

Moreover, both the upright post and the cam are structurally asymmetrical.

Moreover, the rear surface of the upright post is gradually extended forwards from outside to inside.

Moreover, outer edges at both ends of the cam have equal diameter.

Moreover, the front side of the upright post adopts the design of a circular sloping shoulder; a front surface of the upright post is a circular surface; and the upright post is in the shape of a thumb with radian on the whole in side view.

Compared with the prior art, in the present disclosure, the first arc surface on the upright post and the third arc surface on the cam cooperate with each other to conveniently guide smooth external rotation of the femur relative to the tibia; the second arc surface on the upright post and the fourth arc surface on the cam cooperate with each other to gradually limit the external rotation of the femur relative to the tibia; and when the second arc surface and the fourth arc surface make full contact with each other, the external rotation of the femur relative to the tibia can be completely stopped. The artificial knee joint provided by the present disclosure adopts an actual means to accurately simulate the flexion and extension of the knee joint, is safe and reliable, can further improve the usage level of the artificial knee joint, and satisfies higher requirements of patients, meanwhile, the product according to the disclosure has a higher market value.

### BRIEF DESCRIPTION OF THE DRAWINGS

FIG. 1 is a schematic structural view of a femoral component in an artificial knee joint provided by the present disclosure;
FIG. 2 is a schematic structural view of a tibial component in the artificial knee joint provided by the present disclosure;
FIG. 3 is a schematic structural view illustrating the use state I of the artificial knee joint provided by the present disclosure;
FIG. 4 is a schematic structural view illustrating the use state II of the artificial knee joint provided by the present disclosure;
FIG. 5 is a schematic structural view illustrating the use state III of the artificial knee joint provided by the present disclosure;
FIG. 6 is a schematic structural side view of an upright post in the artificial knee joint provided by the present disclosure;
FIG. 7 is a schematic structural perspective view of the upright post in the artificial knee joint provided by the present disclosure;
FIG. 8 is a schematic structural perspective view of a cam in the artificial knee joint provided by the present disclosure; and
FIG. 9 is a schematic diagram illustrating a juncture between a first arc surface and a second arc surface of the upright post in the artificial knee joint provided by the present disclosure.

### DETAILED DESCRIPTION

Detailed description will be given below to the preferred embodiments which illustrate the characteristics and advantages of the present disclosure. It should be understood that various variations could be made to the present disclosure on the basis of different embodiments without departing from the scope of the present disclosure; and the description and accompanying drawings therein are only for illustration in essence and not intended to limit the present disclosure.

In the present disclosure, the "front" direction and the "rear" direction referred herein are consistent with the fore-and-aft direction of the human body after the artificial knee joint is implanted into the human body. Description will be given to the present disclosure by taking a left artificial knee joint as an example.

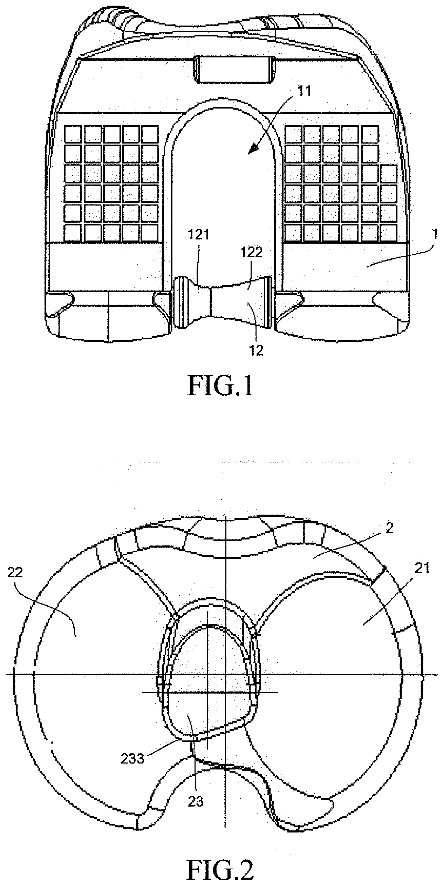

As shown in FIGS. 1 and 2, the artificial knee joint provided by the present disclosure comprises a femoral component 1 and a tibial component 2. The femoral component 1 is fixed at a distant end of a femur, and the tibial component 2 is fixed at a proximal end of a tibia. The femoral component 1 and the tibial component 2 cooperate with each other in such a way that the tibia can support the femur through the artificial knee joint according to the present disclosure. Moreover, the femoral component 1 and the tibial component 2 can also cooperate with each other to simulate the flexion and extension of the knee joint of the human body.

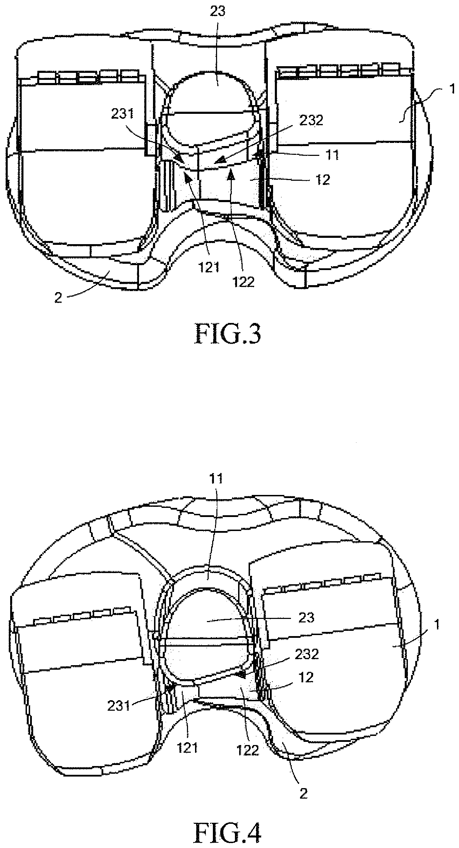

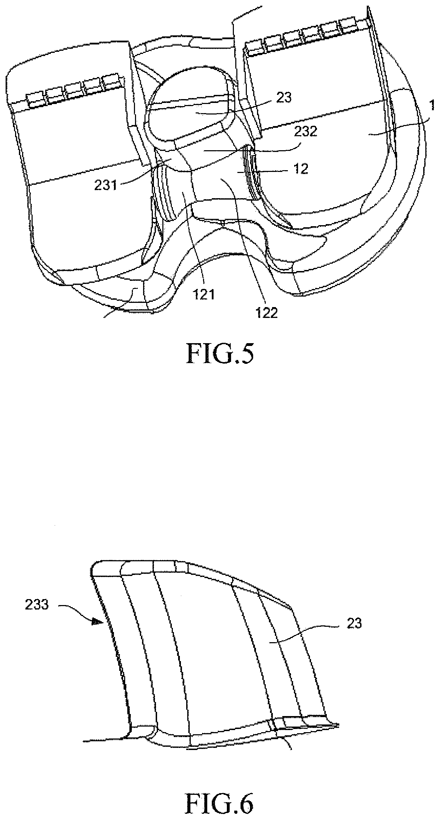

FIGS. 3 to 5 illustrate the cooperation relationship between the femoral component 1 and the tibial component 2, in which a front half part of the femoral component 1 is removed for sake of clearness. As shown in FIGS. 2 and 3, the tibial component 2 is provided with a medial condyle supporting surface 21 and a lateral condyle supporting surface 22 on the right and left sides, respectively. An upright post 23 is disposed between the medial condyle supporting surface 21 and the lateral condyle supporting surface 22 of the tibial component 2, and an intercondylar groove 11 is provided on the femoral component 1. The upright post 23 may be accommodated in the intercondylar groove 11.

As shown in FIGS. 4, 6 and 7, the upright post 23 is in the shape of a thumb 233 with radian on the whole in side view. The thumb 233 with radian can guarantee that the cam 12 cannot be disengaged from the upright post 23 (namely dislocation) when the artificial knee joint provided by the present disclosure performs a deep flexion, and hence the safety in utilization of the artificial knee joint provided by the present disclosure can be guaranteed.

Both a front portion and a rear portion of the upright post 23 have arc surfaces with smooth transition. A width of the front portion of the upright post 23 is less than that of the rear portion. A front side of the upright post 23 adopts the design of a circular sloping shoulder so as to guarantee that the upper front end of the upright post 23 cannot collide with a kneecap when the artificial knee joint provided by the present disclosure is in deep flexion or in the case of low patella, and hence the kneecap can be better protected. Moreover, as a front surface of the upright post 23 is a circular surface, when the upright post 23 makes contact with a circular surface on the front of a femur box at the intercondylar groove 11 of the femoral component 1, the contact area can be increased and the stress can be reduced.

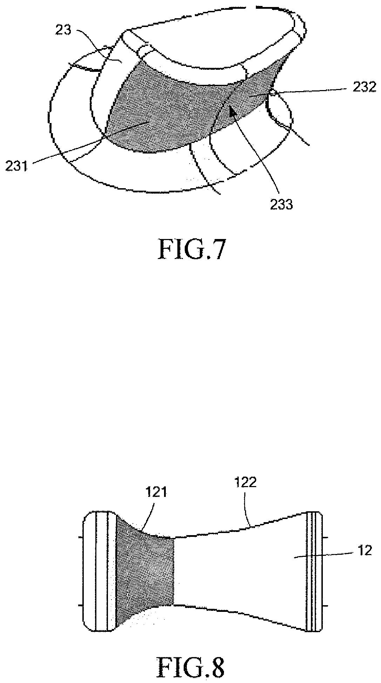

A cam 12 is disposed at the rear of the intercondylar groove 11. As shown in FIG. 8, the cam 12 has a structure in which both ends are large and the central section is small. Diameters of outer edges at both ends of the cam 12 are preferably equal to each other and may also be unequal. No matter in which case, the implementation of the technical solution of the present disclosure cannot be affected.

As shown in FIGS. 3 to 8, in view of the cross section, a first arc surface 231 is formed on the outside of a rear surface of the upright post 23; correspondingly, a third arc surface 121 cooperating with the first arc surface 231 is formed on the outside of a front surface of the cam 12. The first arc surface 231 and the third arc surface 121 may be elliptical surfaces or circular surfaces. A curvature of the first arc surface 231 should be approximately equal to, and most preferably equal to, that of the third arc surface 121. In the embodiment, both the first arc surface 231 and the third arc surface 121 are circular surfaces, and have the same curvature radius of 3 to 10 mm and, most preferably, 5 mm.

As shown in FIGS. 3 to 8, in view of the cross section, a second arc surface 232 is formed on the rear surface of the upright post 23, disposed on the inside of the first arc surface 231. Correspondingly, a fourth arc surface 122 cooperating with the second arc surface 232 is formed on the inside of the front surface of the cam 12. The second arc surface 232 and the fourth arc surface 122 may be elliptical surfaces or circular surfaces; and curvatures of the second arc surface 232 and the fourth arc surface 122 should be proximal and, most preferably, equal. In the embodiment, both the second arc surface 232 and the fourth arc surface 122 are circular surfaces, and have the same curvature radius of 30 to 50 mm and, most preferably, 40 mm.

In the present disclosure, in view of the cross section, the curvatures of the second arc surface 232 and the fourth arc surface 122 are preferably less than those of the first arc surface 231 and the third arc surface 121, namely the second arc surface 232 and the fourth arc surface 122 are more flat than the first arc surface 231 and the third arc surface 121. Therefore, the external rotation of the femur relative to the tibia can be better limited.

When the artificial knee joint provided by the present disclosure is applied to the human body, the cooperation between the femoral component 1 and the tibial component 2 is described as follows.

The knee joint takes the inside as a rotation center and the outside as the rotation locus during the flexion and extension. When the knee joint is flexed and extended, the upright post 23 on the tibial component 2 moves in the intercondylar groove 11 on the femoral component 1. The upright post 23 on the tibial component 2 will make contact with the cam 12 on the femoral component 1 only when the knee joint is flexed to a certain angle.

When the upright post 23 makes contact with the cam 12, the first arc surface 231 of the upright post 23 makes contact with the third arc surface 121 of the cam 12 at first. The two arc surfaces cooperate with each other to guide the external rotation of the femur relative to the tibia, so as to simulate the external rotation of the femur relative to the tibia when the real knee joint of the human body is flexed and extended.

As the continuous flexion of the knee joint, the second arc surface 232 of the upright post 23 gradually makes contact with the fourth arc surface 122 of the cam 12. The two arc surfaces 232 and 122 cooperate with each other to gradually limit the external rotation of the femur relative to the tibia. Moreover, when the two arc surfaces make full contact with each other, the external rotation of the femur relative to the tibia can be completely stopped.

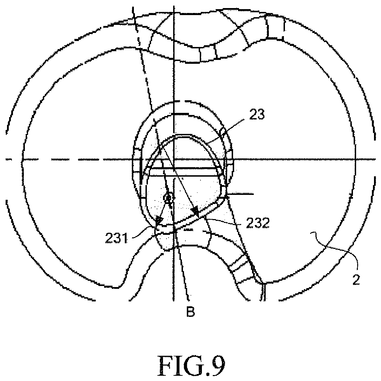

As shown in FIG. 9, the upright post 23 is disposed on the tibial component 2. A juncture between the first arc surface 231 and the second arc surface 232 on the rear surface of the upright post 23 is preferably disposed on the left rear side of the upright post 23, and is preferably disposed at such a position that in the cross section of the tibial component 2, an included angle between a straight line, which connects a center of a circle of the first arc surface 231 and the juncture of the first and second arc surfaces 231 and 232, and a straight line, which is in the fore-and-aft direction of the tibia, is 5 to 20 degrees and, most preferably, 10 to 15 degrees. As seen from the cooperation between the femoral component 1 and the tibial component 2, such angle is the angle of external rotation of the femur relative to the tibia.

As shown in FIG. 2, the upright post 23 is asymmetrically disposed on the tibial component 2 and is disposed on a postmedial portion of the tibial component 2, biased towards the lateral condyle direction (center positions of the upright post 23 and the tibial component 2 are shown by a cross line in the figure), namely biased towards the lateral condyle supporting surface 22. The eccentricity of the upright post 23 relative to the tibial component 2 is preferably disposed such that the upright post 23 is biased afterwards 0.5 to 5 mm and biased outwards 0.5 to 5 mm. The eccentricity may be determined by different conditions of the human bodies. The arrangement mode of the asymmetrical position of the upright post 23 relative to the tibial component 2 is closer to the normal anatomy of the human body and is more advantageous to guide and limit the external rotation of the femur relative to the tibia by the artificial knee joint provided by the present disclosure.

Moreover, the upright post 23 and the cam 12 may be arranged to have structures with symmetrical shapes and dimensions according to actual requirements, but are preferably arranged to be structurally asymmetrical, because the asymmetrical structures can more easily satisfy the above functional requirements. The rear surface of the upright post 23 may be asymmetrical and preferably gradually extended forwards from outside to inside. Meanwhile, the front surface of the upright post 23 may also be asymmetrical. In the present disclosure, the upright post 23 preferably has an asymmetrical structure on the whole. Correspondingly, the cam 12 may only has an asymmetrical front surface, but preferably, the cam 12 has an asymmetrical structure on the whole.

The artificial knee joint provided by the present disclosure has advantages below: compared with the prior art, as the first arc surface 231 on the upright post 23 and the third arc surface 121 on the cam 12 cooperate with each other, smooth external rotation of the femur relative to the tibia can be conveniently guided; as the second arc surface 232 on the upright post 23 and the fourth arc surface 122 on the cam 12 cooperate with each other, the external rotation of the femur relative to the tibia can be gradually limited; and when the second arc surface 232 makes full contact with the fourth arc surface 122, the external rotation of the femur relative to the tibia can be completely stopped. The artificial knee joint provided by the present disclosure adopts an actual means to accurately simulate the flexion and extension of the knee joint, is safe and reliable, can further improve the usage level of the artificial knee joint, satisfies higher requirements of patients, and meanwhile has a higher market value.

The technical solutions of the present disclosure have been described above with reference to the preferred embodiments. It should be understood by those skilled in the art that all the modifications and variations made without departing from the scope of the invention as defined by the appended claims should fall within the scope of protection of the present disclosure.

## Claims

1. An artificial knee joint comprising a femoral component (1) and a tibial component (2) cooperating with each other and applied to the femur and the tibia, respectively, wherein
the femoral component (1) is provided with an intercondylar groove (11), and a cam (12) is disposed at the rear of the intercondylar groove (11);
the tibial component (2) is provided with a medial condyle supporting surface (21) and a lateral condyle supporting surface (22) disposed on the left and right sides, respectively; and
an upright post (23) is disposed between the medial condyle supporting surface (21) and the lateral condyle supporting surface (22) and cooperates with the intercondylar groove (11), **characterized in that**
a first arc surface (231) is formed on the outside of a rear surface of the upright post (23); and a second arc surface (232) is formed on the rear surface of the upright post (23), on an inside of the first arc surface (231 ), wherein the first arc surface has a different curvature to the second arc surface; the cam (12) being correspondingly provided with a third arc surface (121) and a fourth arc surface (122) cooperating with the first arc surface (231) and the second arc surface (232), respectively, wherein the third arc surface has a different curvature to the fourth arc surface; whereby the first arc surface (231) can cooperate with the third arc surface (121) to guide an external rotation of the femur relative to the tibia; and
the second arc surface (232) can cooperate with the fourth arc surface (122) to limit the external rotation of the femur relative to the tibia.

2. The artificial knee joint according to claim 1, **characterized in that**
in view of the cross section, the curvature of the first arc surface (231) is the same as that of the third arc surface (121);
the curvature of the second arc surface (232) is the same as that of the fourth arc surface (122); and
the curvature of the second arc surface (232) and the fourth arc surface (122) is less than that of the first arc surface (231) and the third arc surface (121).

3. The artificial knee joint according to claim 2, **characterized in that** the first arc surface (231), the second arc surface (232), the third arc surface (121) and the fourth arc surface (122) are all circular surfaces.

4. The artificial knee joint according to claim 3, **characterized in that** the radius of the first arc surface (231) and the third arc surface (121) is 3 to 10 mm; and the radius of the second arc surface (232) and the fourth arc surface (122) is 30 to 50 mm.

5. The artificial knee joint according to claim 3, **characterized in that**, in the cross section of the tibial component (2), an included angle between a straight line, which connects a center of a circle of the first arc surface (231) and a juncture of the first arc surface (231) and the second arc surface (232), and a straight line, which is in the fore-and-aft direction of the tibia, is 5 to 20 degrees.

6. The artificial knee joint according to claim 1, **characterized in that** the upright post (23) is asymmetrically disposed on the tibial component (2) and is disposed on a postmedial portion of the tibial component (2), biased towards the lateral condyle supporting surface (22).

7. The artificial knee joint according to claim 1, **characterized in that** both the upright post (23) and the cam (12) are structurally asymmetrical.

8. The artificial knee joint according to claim 7, **characterized in that** the rear surface of the upright post (23) is gradually extended forwards from outside to inside.

9. The artificial knee joint according to claim 7, **characterized in that** outer edges at both ends of the cam (12) have an equal diameter.

10. The artificial knee joint according to claim 1, **characterized in that**
the front side of the upright post (23) adopts the design of a circular sloping shoulder; a front surface of the upright post (23) is a circular surface; and
the upright post (23) is in the shape of a thumb with radian on the whole in side view.

## Patentansprüche

1. Ein künstliches Kniegelenk, das eine femorale Komponente (1) und eine tibiale Komponente (2) aufweist, welche miteinander zusammenwirken und an dem Femur beziehungsweise an der Tibia appliziert sind, wobei
die femorale Komponente (1) mit einer interkondylären Nut (11) versehen ist und ein Nocken (12) am hinteren Teil der interkondylären Nut (11) angeordnet ist;
die tibiale Komponente (2) mit einer medialen Kondylus-Stützfläche (21) und einer seitlichen Kondylus-Stützfläche (22) auf der rechten beziehungsweise der linken Seite versehen ist; und
ein aufrechter Stiel (23) zwischen der medialen Kondylus-Stützfläche (21) und der seitlichen Kondylus-Stützfläche (21) angeordnet sind und mit der interkondylären Nut (11) zusammenwirkt, **dadurch gekennzeichnet, dass**
eine erste Bogenfläche (231) an der Außenseite einer rückseitigen Fläche des aufrechten Stiels (23) ausgebildet ist;
und
eine zweite Bogenfläche (232) an der Rückseite des aufrechten Stiels (23) an einer Innenseite der ersten Bogenfläche (231) ausgebildet ist, wobei die erste Bogenfläche eine von der zweiten Bogenfläche verschiedene Krümmung aufweist;
wobei der Nocken (12) entsprechend mit einer dritten Bogenfläche (121) und einer vierten Bogenfläche (122) versehen ist, die mit der ersten Bogenfläche (121) beziehungsweise der zweiten Bogenfläche (232) zusammenwirken, wobei die dritte Bogenfläche eine von der vierten Bogenfläche verschiedene Krümmung aufweist; wobei
die erste Bogenfläche (231) mit der dritten Bogenfläche (121) zusammenwirken kann, um eine Außendrehung des Femurs relativ zu der Tibia zu führen; und
die zweite Bogenfläche (232) mit der vierten Bogenfläche (122) zusammenwirken kann, um die Außendrehung des Femurs relativ zu der Tibia zu begrenzen.

2. Künstliches Kniegelenk nach Anspruch 1, **dadurch gekennzeichnet, dass**
im Hinblick auf den Querschnitt die Krümmung der ersten Bogenfläche (231) die gleiche wie die der dritten Bogenfläche (121) ist;
die Krümmung der zweiten Bogenfläche (232) die gleiche wie die der vierten Bogenfläche (122) ist; und
die Krümmung der zweiten Bogenfläche (232) und der vierten Bogenfläche (122) geringer als die der ersten Bogenfläche (231) und der dritten Bogenfläche (121) ist.

3. Künstliches Kniegelenk nach Anspruch 2, **dadurch gekennzeichnet, dass** die erste Bogenfläche (231), die zweite Bogenfläche (232), die dritte Bogenfläche (121) und die vierte Bogenfläche (122) alle kreisförmigen Flächen sind.

4. Künstliches Kniegelenk nach Anspruch 3, **dadurch gekennzeichnet, dass** der Radius der ersten Bogenfläche (231) und der dritten Bogenfläche (121) 3 bis 10 mm beträgt; und der Radius der zweiten Bogenfläche (232) und der vierten Bogenfläche (122) 30 bis 50 mm beträgt.

5. Künstliches Kniegelenk nach Anspruch 3, **dadurch gekennzeichnet, dass** im Querschnitt der tibialen Komponente (2) ein eingeschlossener Winkel zwischen einer geraden Linie, die eine Mitte eines Kreises der ersten Bogenfläche (231) und eine Verbindungsstelle der ersten Bogenfläche (231) und der zweiten Bogenfläche (232) verbindet, und einer geraden Linie, die in der Vor-und-Zurück-Richtung der Tibia liegt, 5 bis 20 Grad beträgt.

6. Künstliches Kniegelenk nach Anspruch 1, **dadurch gekennzeichnet, dass** der aufrechte Stiel asymmetrisch an der tibialen Komponente (2) angeordnet ist und an einem zu der seitlichen Kondylus-Stützfläche (22) vorgespannten, postmedialen Abschnitt der tibialen Komponente (2) angeordnet ist.

7. Künstliches Kniegelenk nach Anspruch 1, **dadurch gekennzeichnet, dass** der aufrechte Stiel (23) und der Nocken (12) strukturell asymmetrisch sind.

8. Künstliches Kniegelenk nach Anspruch 7, **dadurch gekennzeichnet, dass** die hintere Fläche des aufrechten Stiels (23) graduell vorwärts von außen nach innen verlängert wird.

9. Künstliches Kniegelenk nach Anspruch 7, **dadurch gekennzeichnet, dass** äußere Kanten an beiden Enden des Nockens (12) einen gleichen Durchmesser haben.

10. Künstliches Kniegelenk nach Anspruch 1, **dadurch gekennzeichnet, dass**
die Vorderseite des aufrechten Stiels (23) die Gestalt einer kreisförmig schrägen Schulter annimmt; eine vordere Fläche des aufrechten Stiels (23) eine kreisförmige Fläche ist; und
der aufrechte Stiel (23) in einer Seitenansicht in der Gestalt eines Daumens mit Bogenwinkel insgesamt vorliegt.

## Revendications

1. Articulation artificielle du genou comprenant un composant fémoral (1) et un composant tibial (2) coopérant entre eux et appliqués au fémur et au tibia, respectivement, dans laquelle
le composant fémoral (1) est doté d'un sillon intercondylien (11), et une came (12) est disposée à l'arrière du sillon intercondylien (11) ;
le composant tibial (2) est doté d'une surface de support de condyle interne (21) et d'une surface de support de condyle externe (22) disposées sur les côtés gauche et droit, respectivement ; et
un montant vertical (23) est disposé entre la surface de support de condyle interne (21) et la surface de support de condyle externe (22) et coopère avec le sillon intercondylien (11), **caractérisée en ce que**
une première surface en arc (231) est formée à l'extérieur d'une surface arrière du montant vertical (23) ; et
une deuxième surface en arc (232) est formée sur la surface arrière du montant vertical (23), sur une partie interne de la première surface en arc (231), où la première surface en arc présente une courbure différente de celle de la deuxième surface en arc ;
la came (12) étant dotée, de manière correspondante, d'une troisième surface en arc (121) et d'une quatrième surface en arc (122) coopérant avec la première surface en arc (231) et la deuxième surface en arc (232), respectivement, où la troisième surface en arc a une courbure différente de celle de la quatrième surface en arc ; moyennant quoi
la première surface en arc (231) peut coopérer avec la troisième surface en arc (121) pour guider une rotation externe du fémur par rapport au tibia ; et
la deuxième surface en arc (232) peut coopérer avec la quatrième surface en arc (122) pour limiter la rotation externe du fémur par rapport au tibia.

2. Articulation artificielle du genou selon la revendication 1, **caractérisée en ce que**
compte tenu de la section transversale, la courbure de la première surface en arc (231) est la même que celle de la troisième surface en arc (121) ;
la courbure de la deuxième surface en arc (232) est la même que celle de la quatrième surface en arc (122) ; et
la courbure de la deuxième surface en arc (232) et de la quatrième surface en arc (122) est inférieure à celle de la première surface en arc (231) et de la troisième surface en arc (121).

3. Articulation artificielle du genou selon la revendication 2, **caractérisée en ce que** la première surface en arc (231), la deuxième surface en arc (232), la troisième surface en arc (121) et la quatrième surface en arc (122) sont toutes des surfaces circulaires.

4. Articulation artificielle du genou selon la revendication 3, **caractérisée en ce que** le rayon de la première surface en arc (231) et de la troisième surface en arc (121) est de 3 à 10 mm ; et
le rayon de la deuxième surface en arc (232) et de la quatrième surface en arc (122) est de 30 à 50 mm.

5. Articulation artificielle du genou selon la revendication 3, **caractérisée en ce que**, dans la section transversale du composant tibial (2), un angle inclus entre une ligne droite qui relie un centre d'un cercle de la première surface en arc (231) et une jonction de la première surface en arc (231) et de la deuxième surface en arc (232), et une ligne droite qui est dans la direction longitudinale du tibia, est de 5 à 20 degrés.

6. Articulation artificielle du genou selon la revendication 1, **caractérisée en ce que** le montant vertical (23) est disposé de manière asymétrique sur le composant tibial (2) et est disposé sur une partie post-médiale du composant tibial (2), sollicité vers la surface de support de condyle externe (22).

7. Articulation artificielle du genou selon la revendication 1, **caractérisée en ce que** le montant vertical (23) et la came (12) sont tous deux structurellement asymétriques.

8. Articulation artificielle du genou selon la revendication 7, **caractérisée en ce que** la surface arrière du montant vertical (23) s'étend graduellement vers l'avant de l'extérieur vers l'intérieur.

9. Articulation artificielle du genou selon la revendication 7, **caractérisée en ce que** les bords extérieurs au niveau des deux extrémités de la came (12) ont un diamètre égal.

10. Articulation artificielle du genou selon la revendication 1, **caractérisée en ce que**
le côté avant du montant vertical (23) adopte la conception d'un épaulement incliné circulaire ; une surface avant du montant vertical (23) est une surface circulaire ; et
le montant vertical (23) présente la forme d'un pouce à radian globalement en vue latérale.
